# FASCICULE DE BREVET EUROPEEN

(11) **EP 2 382 449 B1**
(45) Date de publication et mention de la délivrance du brevet: **20.11.2019**
(21) Numéro de dépôt: 10704583.3
(22) Date de dépôt: 19.01.2010
(51) Int. Cl.: G01F 1/66, G01F 1/708, F04B 19/00, A61M 5/142, A61M 5/168

(54) **PROCÉDÉ DE MESURE ET BANC DE MESURE PORTABLE DE MICRO-DÉBITS DE LIQUIDE, APPLICATION À LA CARACTÉRISATION DE MICRO-POMPES À USAGE MÉDICAL**
MESSVERFAHREN UND TRAGBARE MESSBANK FÜR FLÜSSIGE MIKROSTRÖME UND ANWENDUNG ZUR CHARAKTERISIERUNG VON MIKROPUMPEN ZUR MEDIZINISCHEN VERWENDUNG
MEASURING METHOD AND PORTABLE MEASURE BENCH FOR LIQUID MICRO-FLOWS, AND APPLICATION FOR CHARACTERISING MICRO-PUMPS FOR MEDICAL USE

(30) Priorité: 23.01.2009 FR 0900297
(43) Date de publication de la demande: 02.11.2011
(73) Titulaire: Institut National des Sciences Appliquées de Toulouse, 31077 Toulouse Cedex 4 (FR); Université Paul Sabatier, 31062 Toulouse Cedex 4 (FR); Sadir Assistance, 31670 Labege (FR); Federation Antadir, 75006 Paris (FR)
(72) Inventeur: COLIN, Stéphane, F-31400 Toulouse (FR); BARROT, Christine, F-31100 Toulouse (FR); LAURIEN, Nicolas, F-31400 Toulouse (FR); BALDAS, Lucien, F-31320 Castanet (FR); FORET, Didier, F-75013 Paris (FR); CASANOVA, Frédéric, F-31280 Aigrefeuille (FR); THUILLART, Olivier, F-91820 Boutiggny sur Essonne (FR)
(74) Mandataire: Novagraaf Technologies
(86) Numéro de dépôt international: PCT/FR2010/000049
(87) Numéro de publication internationale: WO 2010/084268

(56) Documents cités:
- DE-A1-102006 000 430
- JP-A- 62 233 721
- US-A- 4 559 831
- US-A- 5 355 735

## Description

La présente invention se rapporte à un procédé de mesure de micro-débits de liquide, et un banc de mesure portable à cette fin. Ces micro-débits peuvent descendre jusqu'à environ 5×10⁻¹³ m³s⁻¹. L'application principale, mais non exclusive, se rapporte à la caractérisation de micro-pompes à usage médical, en particulier aux pompes de délivrance d'insuline, notamment en débit pédiatrique, à partir de mesures de micro-débits.

Le domaine concerné est celui de la métrologie microfluidique et plus particulièrement de la microdébitmétrie.

Outre l'application à la caractérisation débitmétrique des micro-pompes, notamment à usage médical, et plus particulièrement des micro-pompes à insuline, d'autres systèmes peuvent bénéficier de l'analyse de micro-débits selon l'invention: laboratoires d'analyse sur puces, micro-échangeurs thermiques, micro-valves, analyse de micro-écoulements à des fins de recherche fondamentale, etc.

Afin de réaliser des mesures de micro-débits, il est par exemple connu du document intitulé « mise au point et validation d'un banc d'essais pour la mesure de microdébits liquides », présenté par Anduze et al in 14ème Congrès Français de Mécanique, CDROM ISBN 2-84088-040-7. Toulouse, 1999, un banc d'essai pour la mesure de microdébits de liquides dans la plage 10⁻¹³ à 10⁻⁸ m³s⁻¹. Ce document décrit une double mesure effectuée à l'amont et à l'aval du microsystème testé, qui peut être actif (micropompe, microvalve,...) ou passif (microclapet, microcanal,...). Dans le cas de microsystèmes passifs, les pressions amont et aval sont réglées de façon indépendante dans une gamme allant de 0 à 0,5 MPa. Le principe de mesure est basé sur une détection de passage de bulle dans des pipettes étalons, à l'aide de capteurs optiques.

Par ailleurs, le document de brevet US 6 813 944 décrit un débitmètre thermique qui consiste à exploiter les échanges thermiques au sein de l'écoulement d'un fluide forcé à traverser un capillaire. Une résistance chauffante dont la température est supérieure à celle de l'écoulement est placée dans celui-ci. Un transfert thermique donne alors lieu à un gradient de température, mesuré à l'aide de capteurs en amont et en aval de l'élément chauffant. La différence de température est alors proportionnelle au débit massique. Des débits pouvant aller jusqu'à 10⁻¹² m³s⁻¹ peuvent être mesurés.

Cependant, les documents connus ne permettent pas de mesurer de micro-débits de pompes de type pompe à insuline tout en suivant leur fonctionnement pas à pas, afin de mettre en évidence les irrégularités de leur débit. Or, il est essentiel de réaliser des mesures de manière quasi-instantanée dans des conditions atmosphériques variables, en température et en pression. Ces micro-pompes peuvent de plus être confrontées à une surpression au niveau de l'injection du médicament et doivent alors pouvoir se mettre en position de sécurité (arrêt de la pompe) au-delà d'une certaine valeur de cette pression, afin d'éviter les problèmes d'occlusion. Il est donc important de pouvoir mesurer en temps réel les irrégularités de débit et de suivre ces caractéristiques de surpression.

D'autres documents de l'état de la technique, par exemple US 5 355 735 A, US 4 559 831 A, EP 649 341, GB 2 119 927 ou US 4 932 250, qui concernent des dispositifs de mesures d'écoulement ou de micro-débits, ne décrivent pas davantage de moyens de mesure quasi-instantanée de micro-débits, mettant en évidence les irrégularités de débit.

Les solutions connues nécessitent en général une longue attente pour chaque point de mesure du fait de la stabilité thermique des bancs. Pour les plus faibles débits, par exemple dans le cas d'un débit pédiatrique d'insuline, la mesure elle-même peut demander jusqu'à 150 heures.

L'invention propose de fournir un procédé et un banc de mesure portable permettant de pallier aux insuffisances décrites ci-dessus.

Pour ce faire, l'invention prévoit un banc couplant deux moyens de mesure complémentaires, afin de détecter un éventuel dysfonctionnement et de fournir une mesure moyenne et une mesure instantanée du débit. La mesure quasi-instantanée résulte d'un suivi par visualisation continue d'une bulle dans un micro-capillaire ; elle est compatible avec un banc de haute compacité, donc portable.

Plus précisément, l'invention a pour objet un procédé de mesure de micro-débits de liquide d'une micro-pompe dans un caisson selon la revendication 1.

Selon des modes de réalisation avantageux :
- la contre-pression à laquelle doit faire face la pompe est réglable ;
- la contre-pression maximale à ne pas dépasser est égale à la pression d'occlusion mesurée directement en sortie de pompe
- le caisson est régulée en température et en pression atmosphérique en fonction des conditions environnementales d'utilisation.

L'invention se rapporte également à un banc de mesure portable de micro-débits de liquide délivré par une micro-pompe selon la revendication 5.

Selon des modes de réalisation particuliers :
- la contre-pression à laquelle la pompe est soumise en utilisation est ajustée en sortie de la micro-pipette par une commande fournie par l'unité de traitement à une ouverture variable, en particulier une vanne à pointeau ;
- la pompe est connectée sur un plot de branchement apte à mesurer directement la pression d'occlusion par un capteur spécifique en bloquant le débit de sortie jusqu'à amener la pompe en arrêt automatique de sécurité ;
- le plot d'injection est équipé d'un capteur de pression dédié à la mesure de la contre-pression à laquelle la pompe doit faire face, ci-après contre-pression de pompe ;
- le banc est disposé dans un caisson étanche et isolée thermiquement, comportant des moyens de contrôle en température et pression ;
- le moyen de contrôle en température est un module à effet Peltier, disposé dans le caisson, et qui est apte à réguler la température ;
- la micropipette est en verre ;
- le gaz formant la micro-bulle est constitué d'air.

L'invention se rapporte à la caractérisation de micro-pompes et en particulier à la caractérisation de micro-pompes à insuline, dans des conditions de pression et/ou de température variables, le caisson pouvant être dépressurisé.

La présente invention présente les avantages suivants :
- le suivi personnalisé du fonctionnement pas à pas de chaque micro-pompe permet de détecter les irrégularités de fonctionnement de la micro-pompe de manière quasi-instantanée ;
- les mesures de débits moyens sont effectuées sur une période pouvant s'étendre jusqu'à une journée ou plus ;
- la pompe peut être testée dans des conditions de température contrôlée ;
- la pompe peut être testée et son fonctionnement analysé dans des conditions de température et/ou de pression atmosphérique variable, par exemple dans un environnement froid ou pour simuler son utilisation dans un aéronef ;
- le plot d'insertion automatique permet de programmer et de réaliser de façon automatique une campagne de mesures complète, en particulier dans différentes conditions de température ;
- l'analyse d'une micro-pompe dans différentes configurations de fonctionnement peut ainsi être totalement automatisée ;
- l'utilisation d'une micropipette et d'un suivi optique de la bulle ne génère pas de perte de charge non désirée et ne perturbe pas le fonctionnement de la micro-pompe testée ;
- aucun étalonnage en fonction du fluide n'est nécessaire, les mesures de débit volumique étant directes ;
- la fiabilité des mesures et leur précision sont optimisées par le couplage de deux moyens de mesures complémentaires, la sécurité étant ainsi assurée dans le cas d'usage médical ;
- le caractère portable du banc d'essai, rendu possible par les conditions de mise en œuvre de moyens adaptés aux valeurs très faibles de débits à mesurer, permet une utilisation hors laboratoire spécialisé.

L'invention sera mieux comprise et d'autres caractéristiques et avantages apparaîtront à la lecture de la description détaillée qui suit, relative à des exemples de réalisation non limitatifs, en référence aux dessins annexés qui représentent, respectivement :
- la figure 1, le schéma d'un exemple de banc de mesure conforme à l'invention ;
- la figure 2, la vue en coupe d'un exemple de plot d'injection de la micro-bulle et de mesure de la contre-pression du banc selon la figure 1; et
- la figure 3, un exemple de système de mesure optique à capteurs et caméra de visualisation.

Pour des questions de clarté, les schémas annexés ne comportent pas les circuits d'alimentation électrique ni leur aménagement qui sont dans le champ de compétence de l'homme du métier.

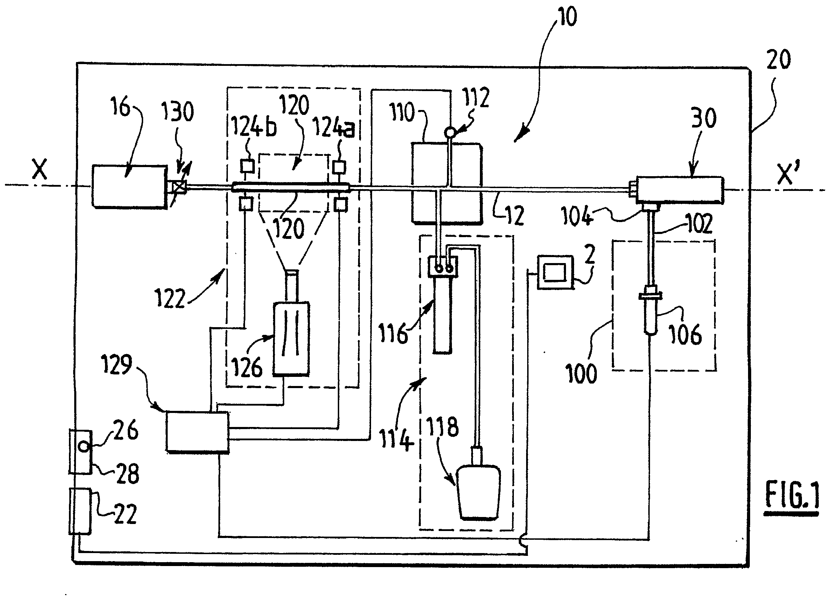

En référence à la figure 1, un banc de mesure 10 selon l'invention est disposé dans une enceinte. Cette enceinte se présente sous la forme d'un caisson 20, isolé thermiquement par le biais de parois doublées de matière isolante. Ce caisson est clos, rendant également l'enceinte étanche.

Le contrôle et la régulation de la température se font par l'intermédiaire d'un module à effet Peltier 22 en liaison avec un capteur de température 24 placés dans l'enceinte 20. La température est homogénéisée au sein de l'enceinte par un ou plusieurs petits ventilateurs. La valeur de la température est contrôlée automatiquement par une boucle de régulation. Un manomètre 26 permet de mesurer la pression dans l'enceinte. La pression désirée est ajustée par une pompe à vide 28.

Dans l'enceinte, la micro-pompe à insuline 30 testée dans l'exemple illustré est montée sur le banc de mesure 10 à une extrémité d'un conduit 12 relié à son autre extrémité à une micro-pipette 120, de diamètre intérieur environ égal à ***60 µm**.* En sortie de la micro-pipette, le liquide est récupéré dans un réservoir 16 après son passage dans le circuit formant le banc 10.

Un capteur 106 de mesure de la pression d'occlusion de pompe est connecté à un embout 102 d'extrémité d'un plot de branchement 100 qui comporte, à son autre extrémité, un embout de branchement universel 104 de pompe, connecté à la micro-pompe 30.

De la micro-pompe 30 au réservoir de récupération 16, le banc 10 reçoit différents composants qui se succèdent successivement à travers :
- un plot d'injection et de mesure 110 sur lequel sont montés un capteur de pression miniaturisé 112, pour la mesure de pression et le réglage ultérieur de la contre-pression, et un système d'injection automatique de bulle 114 comportant une électrovanne 116 et une pompe à air 118 ;
- la micro-pipette en verre 120 située dans un système de mesure optique de débit 122, composé de capteurs optiques 124a et 124b, d'une caméra 126 ayant une zone de visualisation 128 qui intercepte une partie significative de la micro-pipette 120 ;
- un mécanisme de restriction variable 130 en sortie de la micro-pipette 120 pour régler la valeur de la contre-pression, le réservoir 16 étant monté en sortie du mécanisme 130.

Le banc comporte également une unité de traitement des données 129 qui gère les données fournies par les capteurs et la caméra, fournit des signaux de commande et transmet des informations utiles à l'utilisateur, comme cela apparaîtra plus loin.

Le système d'injection automatique 114 comporte l'électrovanne 116 et comme pompe à air 118 une pompe de type pompe d'aquarium. L'actionnement de l'électrovanne 116 est déclenché automatiquement par le passage de la bulle précédente devant le capteur optique 124b placé en sortie de la micro-pipette 120, dans le cas d'une série de mesures consécutives.

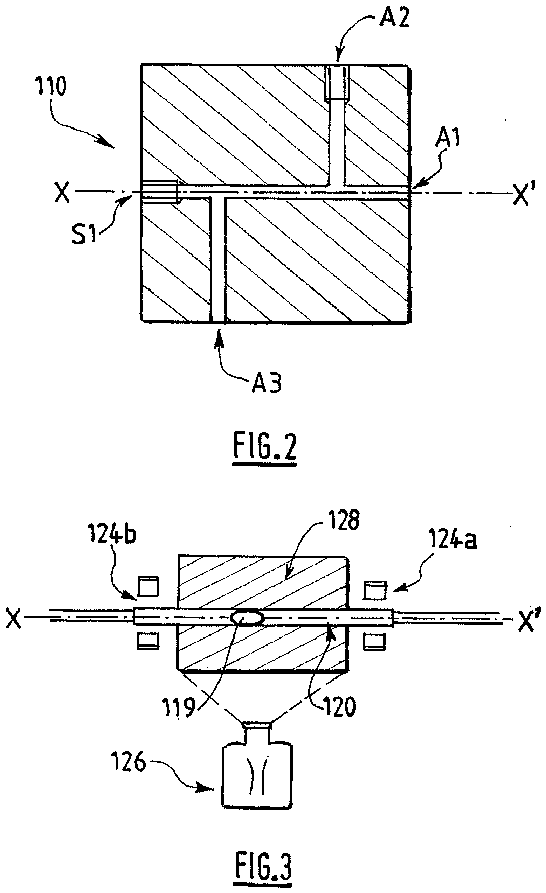

Le plot d'injection et de mesure 110 est plus particulièrement présenté en regard de la figure 2. Ce plot 110 est réalisé de manière à pouvoir connecter tout type de conduit de micro-pompe, en particulier une micro-pompe à insuline, par son entrée A1. Le micro-capteur de pression est connecté à l'orifice A2. La sortie S1 permet de connecter la micropipette en verre. L'orifice A3 est destiné à relier le système d'injection 114, l'écoulement principal de liquide X'X se faisant de A1 vers S1. Ce plot est conçu de façon à contenir un volume de fluide minimal, de l'ordre de ***1.5 µl.***

La pompe à air exerce une pression légèrement supérieure à celle de la conduite principale A1-S1, afin de réaliser l'insertion d'une bulle 119 (figure 3) dans l'écoulement du liquide. La durée d'ouverture de l'électrovanne 116 est réglée de façon à générer une bulle de dimension adaptée à la micro-pipette.

En référence au système de mesure de débit illustré en figure 3, la mesure de débit est alors réalisée par deux voies :
- la détection du passage de la bulle par deux capteurs optiques 124a et 124b, disposés en entrée et en sortie de la micro-pipette 120, dans le sens d'écoulement X'X du fluide ;
- le suivi continu du déplacement de la bulle 119 par analyse d'une séquence d'images acquise par la caméra 126 dans la zone de visualisation 128.

La caméra 126 est, dans l'exemple illustré, une webcaméra dite « webcam » à capteurs CMOS.

En fonctionnement, la régulation thermique de l'enceinte est effectuée sur une plage de 10 à 50°C par le module Peltier, le réglage en température étant commandé sur une interface utilisateur qui affiche la température obtenue dans l'enceinte.

La première injection automatique de bulles 119 est déclenchée lorsque la micro-pipette est remplie de liquide. Un réglage de la durée d'ouverture de l'électrovanne permet d'adapter la taille de la bulle en fonction des caractéristiques de l'électrovanne 116 et de la pompe à air 118, et également du débit de la micro-pompe et de la contre-pression, de manière à obtenir une détection optique et une visualisation fiable pour les mesures de débit dans le système optique 122.

Ainsi, dans le système optique de l'exemple de réalisation, la durée d'écoulement entre les détections d'une bulle par les capteurs optiques 124a et 124b est relevée par l'unité de traitement de données 129, de manière à calculer un débit moyen sur environ 5 µl. La caméra 126 permet une acquisition vidéo instantanée du déplacement de la bulle par analyse des données image par image.

Le réglage de la contre-pression en sortie de la micro-pipette 120 est effectué par un orifice 130 réglable en ouverture, formé par une micro-vanne à pointeau dans l'exemple. La pression fournie par la pompe est mesurée par le capteur 112 avant injection de la bulle et transmise à l'unité de traitement 129, qui émet un signal de commande à la micro-vanne 130, commandée électriquement dans l'exemple de réalisation. La micro-vanne règle alors la pression, dite contre-pression, que doit vaincre l'écoulement de liquide en sortie de la pompe testée. La valeur maximale de contre-pression ne dépasse pas la valeur de pression d'occlusion mesurée en sortie de pompe par le capteur 106 et transmise à l'unité de traitement 129.

La pression d'occlusion est mesurée par le capteur 106 du plot 100 en bloquant le débit de sortie jusqu'à amener la pompe en arrêt automatique de sécurité.

Les valeurs de débits moyens et instantanés calculés par l'unité de traitement permettent de détecter tout dysfonctionnement de la pompe au regard des valeurs attendues.

L'invention n'est pas limitée aux exemples décrits et représentés.

Il est ainsi possible de prévoir plus de deux capteurs optiques ou de prévoir une bulle de gaz autre que de l'air, par exemple d'azote pour éviter tout échange chimique. Par ailleurs, l'application de l'invention peut être étendue à tout type de pompe à tester qui est susceptible de fournir des micro-débits dans des conditions particulières.

En outre, le caisson qui définit l'enceinte peut être réalisé sous forme d'une valise de manière à faciliter le transport du banc optique.

## Revendications

1. Procédé de mesure de micro-débits de liquide d'une micro-pompe (30) dans un caisson (20) étanche, isolé thermiquement et régulé en température et en pression atmosphérique, **caractérisé en ce qu'**il comprend :
- une captation optique du passage d'une micro-bulle (119) en entrée et en sortie d'une micro-pipette (120) ; et en parallèle
- une acquisition vidéo instantanée du déplacement de la micro-bulle (119) dans la micro-pipette (120) par une caméra (126) ;
- un traitement des mesures de captation optique adapté pour mesurer un débit moyen ;
- une analyse des données image par image de l'acquisition vidéo instantanée pour mesurer un débit instantané ; et
- une détection d'un dysfonctionnement de ladite micro-pompe (30) par comparaison du débit moyen et du débit instantané mesurés par rapport à des valeurs prédéterminées.

2. Procédé de mesure de micro-débits selon la revendication 1, comportant un ajustement de contre-pression en sortie des mesures optiques de débit et une détection de pression d'occlusion que la contre- pression ne peut dépasser.

3. Procédé de mesure de micro-débits selon la revendication précédente, dans lequel la pression d'occlusion est mesurée directement en sortie de pompe.

4. Procédé de mesure de micro-débits selon l'une quelconque des revendications 1 à 3, dans lequel le caisson (20) est régulé en température et pression en fonction des conditions environnementales d'utilisation.

5. Banc de mesure portable (10) de micro-débits de liquide délivré par une micro-pompe (30), **caractérisé en ce qu'**il comporte :
- un plot d'injection (110) apte à insérer automatiquement une micro-bulle (119) de gaz dans une micropipette calibrée (120), dans laquelle circule le liquide délivré par la micro-pompe (30) ; et
- une série de capteurs optiques (124a, 124b) adaptés pour suivre le passage de la micro-bulle (119) en entrée et en sortie de la micro-pipette (120) ; et en parallèle
- une caméra (126) adaptée pour imager une zone de visualisation (128) qui intercepte une partie significative de la micro-pipette (120) ;
- une unité de traitement de données (129) apte à fournir des valeurs de mesures de débits volumiques moyens et instantanés à partir des données délivrées par les moyens optiques de détection (124a, 124b, 126), et adaptée pour comparer les débits moyens et instantanés par rapport à des valeurs prédéterminées pour détecter un dysfonctionnement de ladite micro-pompe (30).

6. Banc de mesure portable selon la revendication précédente, dans lequel la pompe est connectée sur un plot de branchement (100) apte à mesurer directement la pression d'occlusion par un capteur spécifique (106) en bloquant le débit de sortie jusqu'à ce que la pompe (30) s'arrête automatiquement en position de sécurité.

7. Banc de mesure portable selon la revendication 5 ou 6, dans lequel le plot d'injection (110) est équipé d'un capteur de pression (112) dédié à la mesure de la contre-pression de la pompe (30).

8. Banc de mesure portable selon l'une quelconque des revendications 5 à 7, dans lequel l'unité de traitement (129) est apte à fournir une commande à une ouverture variable (130) pour ajuster la contre-pression à laquelle la pompe (30) est soumise en utilisation.

9. Banc de mesure portable selon la revendication précédente, dans lequel l'ouverture variable est une micro-vanne à pointeau (130).

10. Banc de mesure portable selon l'une quelconque des revendications 5 à 9, **caractérisé en ce que** le banc (10) est disposé dans un caisson (20) étanche et isolé thermiquement, comportant des moyens de contrôle (22, 24) en température et pression.

11. Banc de mesure portable selon la revendication 10, dans lequel le moyen de contrôle en température est un module à effet Peltier disposé dans le caisson qui permet de réguler la température.

12. Banc de mesure portable selon l'une quelconque des revendications 5 à 11, dans lequel la micropipette est en verre.

13. Banc de mesure portable selon l'une quelconque des revendications 5 à 12, dans lequel le gaz formant la micro-bulle est constitué d'air.

14. Application du banc de mesure portable selon l'une quelconque des revendications 5 à 13, à la caractérisation de micro-pompes par des mesures de micro-débits selon l'une quelconque des revendications 1 à 4.

15. Application selon la revendication précédente à la caractérisation de micro-pompes à insuline, dans des conditions de pression et/ou de température variables et régulées par le caisson (20).

## Patentansprüche

1. Messverfahren für Flüssigkeitsmikrovolumenströme einer Mikropumpe (30) in einem dichten Gehäuse (20), thermisch isoliert und temperaturreguliert und bei Atmosphärendruck, **dadurch gekennzeichnet, dass** es Folgendes umfasst:
- eine optische Erfassung des Durchgangs einer Mikroblase (119) am Eingang und Ausgang einer Mikropipette (120); und parallel dazu
- eine unmittelbare Videoaufnahme der Bewegung der Mikroblase (119) in der Mikropipette (120) durch eine Kamera (126);
- eine Behandlung der Messungen der optischen Erfassung, angepasst, um einen mittleren Volumenstrom zu messen;
- eine Analyse der Bilddaten durch das Bild der unmittelbaren Videoaufnahme zum Messen eines unmittelbaren Volumenstroms; und
- eine Detektion einer Fehlfunktion der Mikropumpe (30) durch Vergleich des gemessenen mittleren Volumenstroms und unmittelbaren Volumenstroms mit vorher festgelegten Werten.

2. Messverfahren von Mikrovolumenströmen nach Anspruch 1, umfassend eine Anpassung des Gegendrucks am Ausgang der optischen Messungen des Volumenstroms und eine Detektion von Okklusionsdruck, den der Gegendruck nicht überschreiten darf.

3. Messverfahren von Mikrovolumenströmen nach dem vorstehenden Anspruch, wobei der Okklusionsdruck direkt am Ausgang der Pumpe gemessen wird.

4. Messverfahren von Mikrovolumenströmen nach einem der Ansprüche 1 bis 3, wobei das Gehäuse (20) in Funktion der Umweltbedingungen bei der Verwendung temperatur- und druckreguliert ist.

5. Tragbare Messbank (10) für durch eine Mikropumpe (30) geförderte Flüssigkeitsmikrovolumenströme, **dadurch gekennzeichnet, dass** sie Folgendes umfasst:
- ein Injektionspad (110), das geeignet ist, automatisch eine Gas-Mikroblase (119) in eine kalibrierte Mikropipette (120) einzubringen, in der die durch die Mikropumpe (30) geförderte Flüssigkeit zirkuliert; und
- eine Reihe optischer Sensoren (124a, 124b), angepasst zum Folgen des Durchgangs der Mikroblase (119) am Eingang und Ausgang der Mikropipette (120); und parallel dazu
- eine Kamera (126), angepasst zum Abbilden einer Visualisierungszone (128), die einen signifikanten Anteil der Mikropipette (120) abfängt;
- eine Datenbehandlungseinheit (129), die geeignet ist, Messwerte von mittleren und unmittelbaren räumlichen Volumenströmen bereitzustellen, ausgehend von durch die optischen Detektionsmittel (124a, 124b, 126) gelieferten Daten, und angepasst zum Vergleichen der mittleren und unmittelbaren Volumenströme mit den vorher festgelegten Werten, um eine Fehlfunktion der Mikropumpe (30) zu detektieren.

6. Tragbare Messbank nach dem vorstehenden Anspruch, wobei die Pumpe mit einem Anschlusspad (100) verbunden ist, das geeignet ist, den Okklusionsdruck direkt durch einen spezifischen Sensor (106) zu messen, indem der Volumenstrom am Ausgang blockiert wird, bis sich die Pumpe (30) automatisch in Sicherheitsposition ausschaltet.

7. Tragbare Messbank nach Anspruch 5 oder 6, wobei das Injektionspad (110) mit einem Drucksensor (112) ausgestattet ist, der der Messung des Gegendrucks der Pumpe (30) gewidmet ist.

8. Tragbare Messbank nach einem der Ansprüche 5 bis 7, wobei die Behandlungseinheit (129) geeignet ist, einer variablen Öffnung (130) einen Befehl zum Anpassen des Gegendrucks, dem die Pumpe (30) bei Verwendung ausgesetzt ist, bereitzustellen.

9. Tragbare Messbank nach dem vorstehenden Anspruch, wobei die variable Öffnung ein Mikronadelventil (130) ist.

10. Tragbare Messbank nach einem der Ansprüche 5 bis 9, **dadurch gekennzeichnet, dass** die Bank (10) in einem dichten und thermisch isolierten Gehäuse (20) angeordnet ist, umfassend Temperatur- und Drucksteuerungsmittel (22, 24).

11. Tragbare Messbank nach Anspruch 10, wobei das Temperatursteuerungsmittel ein in dem Gehäuse angeordnetes Peltiereffektmodul ist, das gestattet, die Temperatur zu regulieren.

12. Tragbare Messbank nach einem der Ansprüche 5 bis 11, wobei die Mikropipette aus Glas ist.

13. Tragbare Messbank nach einem der Ansprüche 5 bis 12, wobei das Gas, das die Mikroblase bildet, aus Luft besteht.

14. Anwendung der tragbaren Messbank nach einem der Ansprüche 5 bis 13 bei der Charakterisierung von Mikropumpen durch Messungen von Mikrovolumenströme nach einem der Ansprüche 1 bis 4.

15. Anwendung nach dem vorstehenden Anspruch bei der Charakterisierung von Insulin-Mikropumpen unter variablen und durch das Gehäuse (20) regulierten Druck- und/oder Temperaturbedingungen.

## Claims

1. Method for measuring liquid micro-flows of a micro-pump (30) in a watertight caisson (20), thermally insulated and regulated in temperature and in atmospheric pressure, **characterised in that** it comprises:
- an optical pick-up of the passage of a micro-bubble (119) at the inlet and at the outlet of a micro- pipette (120); and in parallel
- an instantaneous video acquisition of the displacement of the micro-bubble (119) in the micro- pipette (120) by a camera (126);
- a processing of the optical pick-up measurements suitable for measuring an average flow;
- an analysis of the data image by image from the instantaneous video acquisition in order to measure an instant flow; and
- a detection of a malfunction of said micro-pump (30) by comparison of the average flow and of the instant flow measured with respect to predetermined values.

2. Method for measuring micro-flows according to claim 1, comprising an adjustment in counter-pressure at the outlet of the optical flow measurements and a detection of the clogging pressure that the counter-pressure cannot exceed.

3. Method for measuring micro-flows according to the preceding claim, wherein the clogging pressure is measured directly at the pump outlet.

4. Method for measuring micro-flows according to any of claims 1 to 3, wherein the caisson (20) is regulated in temperature and pressure according to the environmental conditions of use.

5. Portable measuring bench (10) of liquid micro-flows supplied by a micro-pump (30), **characterised in that** it comprises:
- an injection pad (110) suitable for automatically inserting a micro-bubble (119) of gas into a calibrated micro-pipette (120), wherein circulates the liquid supplied by the micro-pump (30); and
- a series of optical sensors (124a, 124b) adapted to follow the passage of the micro-bubble (119) at the inlet and at the outlet of the micro-pipette (120); and in parallel
- a camera (126) adapted to image a viewing area (128) that intercepts a significant portion of the micro- pipette (120);
- a data processing unit (129) able to supply values of average and instant volume flow measurements using the data supplied by the optical means of detection (124a, 124b, 126), and adapted to compare the average and instant flows with respect to predetermined values in order to detect a malfunction of said micro-pump (30).

6. Portable measuring bench according to the preceding claim, wherein the pump is connected on a connection pad (100) suitable for directly measuring the clogging pressure with a specific sensor (106) by blocking the outlet flow until the pump (30) automatically stops in the safety position.

7. Portable measuring bench according to claim 5 or 6, wherein the injection pad (110) is provided with a pressure sensor (112) dedicated to measuring the counter-pressure of the pump (30).

8. Portable measuring bench according to any of claims 5 to 7, wherein the processing unit (129) is suitable for supplying a controlling by a variable opening (130) in order to adjust the counter-pressure at which the pump (30) is subjected to in use.

9. Portable measuring bench according to the preceding claim, wherein the variable opening is a micro- needle valve (130).

10. Portable measuring bench according to any of claims 5 to 9, **characterised in that** the bench (10) is arranged in a watertight caisson (20) and thermally insulated, comprising means for controlling (22, 24) temperature and pressure.

11. Portable measuring bench according to claim 10, wherein the means of controlling temperature is a Peltier effect module arranged in the caisson that allows adjusting the temperature.

12. Portable measuring bench according to any of claims 5 to 11, wherein the micro-pipette is made of glass.

13. Portable measuring bench according to any of claims 5 to 12, wherein the gas that forms the micro-bubble is composed of air.

14. Application of the portable measuring bench according to any of claims 5 to 13, to the characterisation of micro-pumps by measurements of micro-flows according to any of claims 1 to 4.

15. Application according to the preceding claim to the characterisation of insulin micro-pumps, in variable conditions of pressure and/or of temperature and adjusted by the caisson (20).
